# EUROPEAN PATENT SPECIFICATION

(11) **EP 4 304 494 B1**
(45) Date of publication and mention of the grant of the patent: **09.07.2025**
(21) Application number: 22710164.9
(22) Date of filing: 07.03.2022
(51) Int. Cl.: A61B 17/115, A61B 90/00

(54) **SURGICAL STAPLING APPARATUS WITH INTEGRATED VISUALIZATION**
CHIRURGISCHE KLAMMERVORRICHTUNG MIT INTEGRIERTER VISUALISIERUNG
APPAREIL D'AGRAFAGE CHIRURGICAL AVEC VISUALISATION INTÉGRÉE

(30) Priority: 11.03.2021 US 202163159651 P; 31.12.2021 US 202117566757
(43) Date of publication of application: 17.01.2024
(73) Proprietor: Covidien LP, Mansfield, MA 02048 (US)
(72) Inventor: NICHOLAS, David A., North Haven, Connecticut 06473 (US)
(74) Representative: Maschio & Soames IP Ltd
(86) International application number: PCT/IB2022/051997
(87) International publication number: WO 2022/189934

(56) References cited:
- CN-Y- 200 991 270
- US-A1- 2013 284 792
- US-A1- 2019 357 934

## Description

### CROSS-REFERENCE TO RELATED APPLICATION

This application claims the benefit of U.S. Provisional Patent Application No. 63/159,651, filed March 11, 2021.

### TECHNICAL FIELD

The present disclosure relates to surgical stapling apparatus and, more particularly, to surgical stapling apparatus for inspecting staple formation and hemostasis in anastomosis procedures.

### BACKGROUND

Fasteners have traditionally been used to replace suturing when joining various body structures such as, for example, the bowel or bronchus. Surgical stapling apparatus employed to apply these fasteners are generally designed to simultaneously cut and seal tissue to reduce the time and risks involved with anastomosis procedures.

Circular surgical stapling apparatus are employed by surgeons to apply one or more surgical fasteners, e.g., staples or two-part fasteners, to body tissue for the purpose of joining segments of body tissue together and/or for the creation of anastomoses. Circular surgical stapling apparatus generally include an annular fastener cartridge assembly that supports a plurality of annular rows of fasteners, an annular anvil assembly operatively associated with the fastener cartridge assembly which provides a surface against which the fasteners are formed upon a firing of the circular stapling apparatus, and an annular blade for cutting tissue. One challenge in effectuating anastomoses is ensuring proper staple formation and hemostasis without having direct visualization of the surgical site.
CN 200991270 Y discloses a surgical stapling apparatus comrpising a cartridge assembly, a camera assembly coupled to the anvil assembly via a tether.
US 2013/284792 A1 discloses a surgical stapling apparatus including a handle assembly, an elongated shaft extending distally from the handle assembly, a shell assembly containing a plurality of staples arranged in an annular array, and a firing mechanism for advancing staples from the shell assembly. An anvil assembly including a tiltable anvil head movable from an aligned operative position to a tilted position. A camera assembly is operatively coupled to a distal surface of the tiltable anvil head such that an orientation of the camera assembly changes as the anvil head moves from the operative to the tilted position.

### SUMMARY

The present invention is defined by the features of independent claim 1. Further embodiments are provided by the dependent claims.

Other aspects, features, and advantages will be apparent from the description, the drawings, and the claims that follow.

### BRIEF DESCRIPTION OF DRAWINGS

The accompanying drawings, which are incorporated in and constitute a part of this specification, illustrate aspects of the disclosure and, together with a general description of the disclosure given above and the detailed description given below, explain the principles of the disclosure, wherein:
FIG. 1 is a perspective view of a surgical stapling apparatus according to this disclosure, the surgical stapling apparatus including an end effector shown in an unclamped position;
FIG. 2 is an enlarged, perspective view of the indicated area of detail shown in FIG. 1;
FIG. 3 is a side, perspective view, with parts separated, of a distal portion of the surgical stapling apparatus of FIG. 1;
FIG. 4 is a perspective view illustrating the end effector of the surgical stapling apparatus of FIG. 1 disposed within a tissue lumen and in a clamped position;
FIG. 5 is a cross-sectional view of FIG. 4 as taken along section line 5-5 shown in FIG. 4;
FIGS. 6 and 7 are progressive views illustrating the end effector of FIG. 1 collecting image data of an anastomosis effectuated by the surgical stapling apparatus of FIG. 1;
FIG. 8 is a perspective view of another end effector of the surgical stapling apparatus of FIG. 1 shown disposed in a tissue lumen; and
FIG. 9 is a perspective view of yet another end effector of the surgical stapling apparatus of FIG. 1 shown disposed in a tissue lumen.

### DETAILED DESCRIPTION

Aspects of the disclosed surgical stapling apparatus are described in detail with reference to the drawings, in which like reference numerals designate identical or corresponding elements in each of the several views. As commonly known, the term "clinician" refers to a doctor, a nurse, or any other care provider and may include support personnel. Additionally, the term "proximal" refers to the portion of structure that is closer to the clinician and the term "distal" refers to the portion of structure that is farther from the clinician.

In the following description, well-known functions or constructions are not described in detail to avoid obscuring the present disclosure in unnecessary detail.

In general, this disclosure describes a circular surgical stapling apparatus including a camera assembly tethered to an anvil assembly thereof for facilitating inspection of staple formation, hemostasis, and profusion integrity. The camera assembly can be inserted extracorporeally into a tissue lumen (e.g., an intestine, colon) simultaneously with the anvil assembly. The tissue lumen can be purse string closed around the anvil assembly so that an anastomosis can be effectuated with the end effector of the circular surgical stapling apparatus. The tether is configured to position the camera assembly a predetermined distance away from the anvil assembly to facilitate proper viewing of an anastomosis site. Once the anastomosis is complete, the anvil assembly can be moved relative to the anastomosis site (e.g., retracted therethrough) and the camera assembly can be used to wirelessly transmit image data of the exposed fastener line in the anastomosis site. Optical tissue evaluation can also be performed with such image data to determine a level of profusion and provide the clinician with an additional indication of tissue health. Advantageously, the disclosed stapling apparatus is configured to ensure a hemostatic anastomosis with good profusion that is critical to promote healing and prevent leakage of luminal contents into a patient's abdomen, and which also helps to prevent sepsis. Indeed, this disclosure provides a surgical stapling apparatus with an integrated camera assembly that enables a clinician to inspect both sides of an anastomosis without the need to insert an additional endoscope.

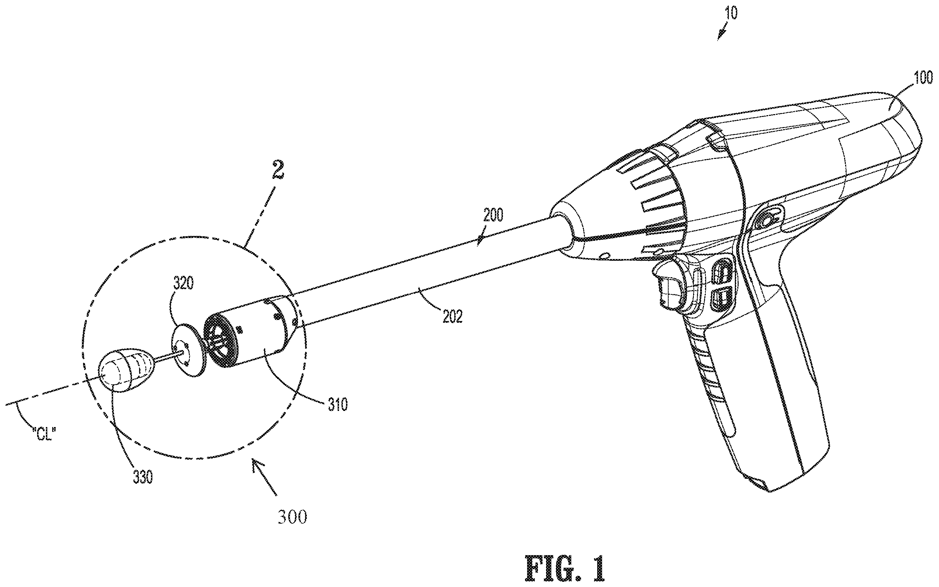

With reference to FIGS. 1-4, a surgical stapling apparatus, generally referred to as 10, is shown. Although shown and described as a circular surgical stapling apparatus, surgical stapling apparatus can have any known suitable surgical stapler configuration (e.g., endoscopic, linear, transverse, and/or open). Moreover, surgical stapling apparatus 10, or portions thereof may be adapted for reuse and, in certain aspects, may be adapted for a single use and/or may be disposable. Surgical stapling apparatus 10 defines a centerline "CL" and includes a surgical device 100 in the form of a powered handheld electromechanical instrument. Surgical stapling apparatus 10 further includes an adapter assembly 200 that is selectively attachable to surgical device 100. Adapter assembly 200 extends distally from surgical device 100 and has an elongated body 202 that extends to a distal end. The distal end of elongated body 202 supports an end effector 300. Surgical device 100 is configured for selective connection with adapter assembly 200, and, in turn, adapter assembly 200 is configured for selective connection with end effector 300. Together, surgical device 100 and adapter assembly 200 cooperate to operate end effector 300.

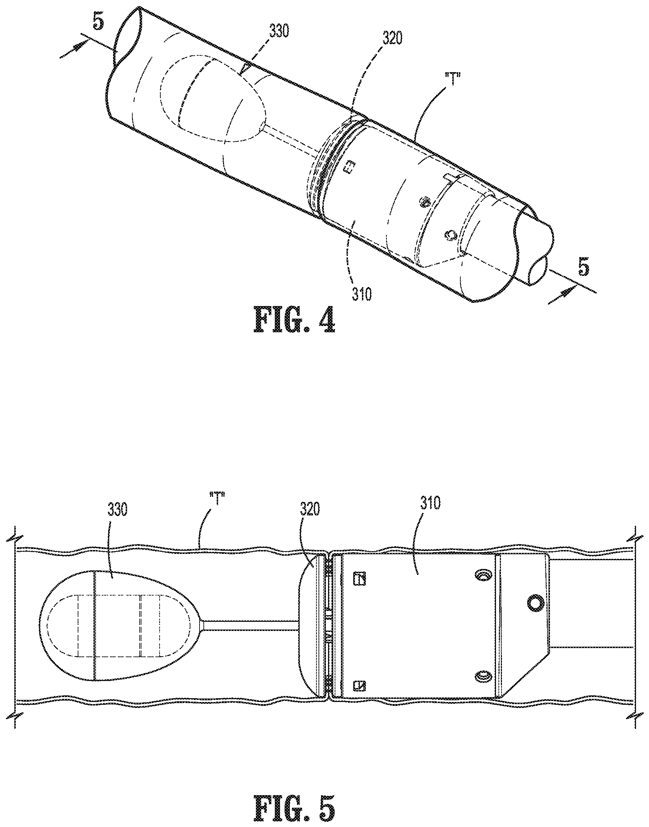

End effector 300 generally includes a shell or cartridge assembly 310 and an anvil assembly 320 that are positionable between an unclamped position (see FIG. 1) and a clamped position (see FIG. 4) to selectively clamp tissue "T" for cutting and/or stapling tissue "T."

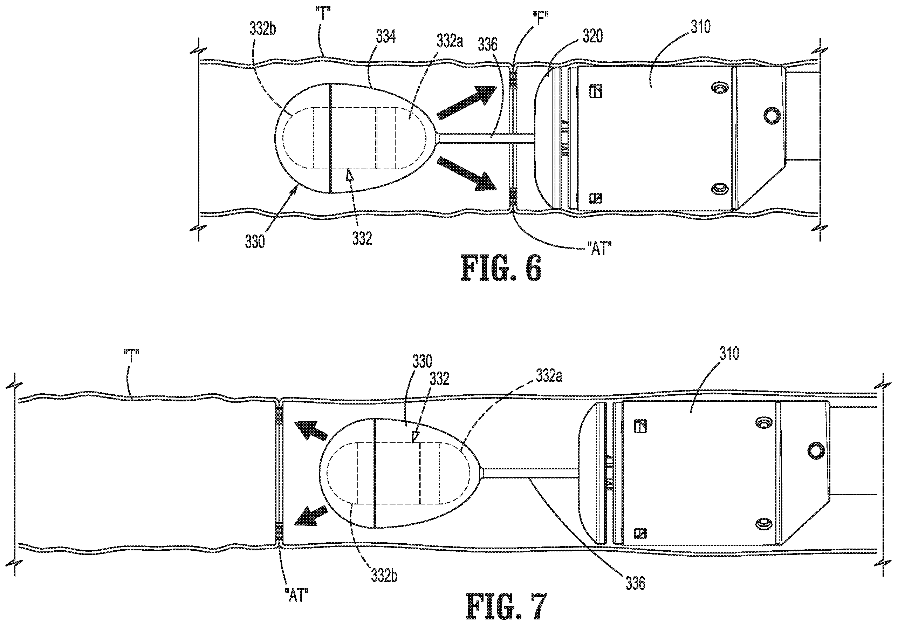

Briefly, cartridge assembly 310 of end effector 300 includes a shell 312 and a staple cartridge 314 that supports fasteners "F" (e.g., staples; see FIG. 6, for example) in an array of fastener slots 316 defined therein. The fasteners "F" are positioned to be fired into anvil assembly 320 for effectuating an anastomosis of tissue "T." Anvil assembly 320 of end effector 300 includes an anvil head 322 and a center rod assembly 324 that extends proximally from anvil head 322. Anvil head 322 defines an array of fastener pockets 323 that form the fasteners "F" when fired from cartridge assembly 310. Center rod assembly 324 is arranged to couple anvil assembly 320 to an anvil retainer 326 extending from cartridge assembly 310 that is movable to move the anvil assembly 320 relative to cartridge assembly 310 between the unclamped and clamped positions.

Reference may be made to U.S. Patent No. 8,806,973 to Ross et al. and U.S. Patent No. 7,303,106 to Milliman et al. for a detailed description of the construction and operation of an example electromechanical surgical stapling apparatus, the components of which are included, combinable and/or interchangeable with one or more components of surgical stapling apparatus 10 described herein.

Although surgical stapling apparatus 10 is described as an electromechanically powered surgical stapling apparatus, the disclosed surgical stapling apparatus 10 can be provided as a manually powered stapling apparatus. For a more detailed description of the construction and operation of an exemplary manually powered stapling apparatus, one or more components of which can be included, combined and/or interchanged with the electromechanically powered stapling apparatus described herein, reference can be made to U.S. Patent No. 5,915,616 to Viola et al., U.S. Patent No. 8,109,426 to Milliman et al., and U.S. Patent No. 8,272,552 to Holsten et al., U.S. Patent No. 9,504,470 to Milliman.

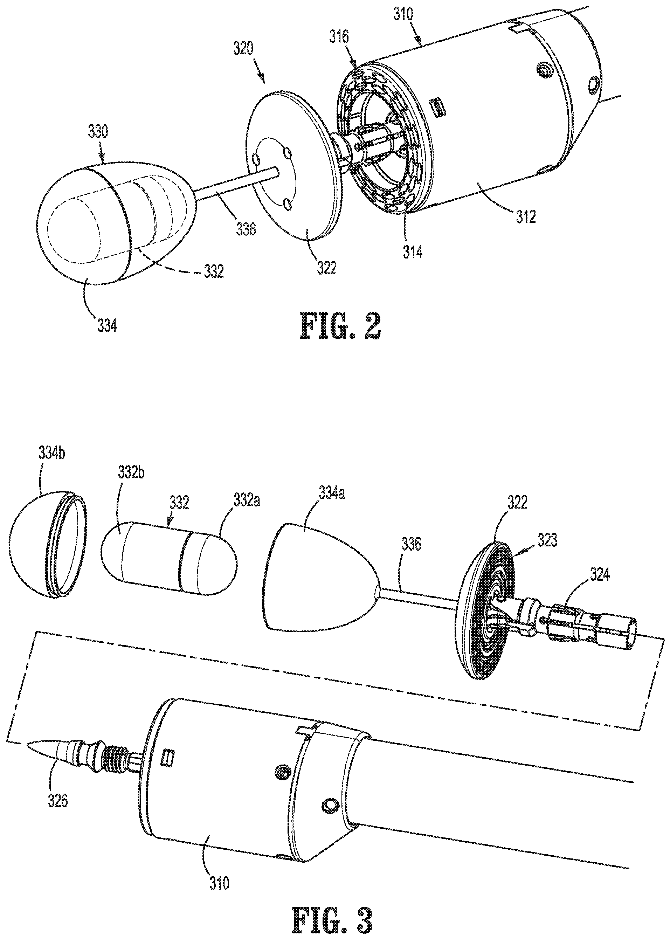

Referring now to FIGS. 2-7, end effector 300 further includes a camera assembly 330 coupled to anvil head 322 of anvil assembly 320. Camera assembly 330 includes a camera member 332, a housing assembly 334 that supports camera member 332, and a tether 336 that secures housing assembly 334 of camera assembly 330 to anvil assembly 320. Tether 336 can be flexible, rigid, and/or semi-rigid to enable end effector 300 to be moved (e.g., distally and/or proximally) to position camera member 332 for optimal viewing. Camera member 332, which is dual-ended, includes a proximal camera 332a that is configured to capture images (e.g., image data of such images) in a proximal direction, and a distal camera 334b that is configured to capture images (e.g., image data of such images) in a distal direction. Each of the proximal and distal cameras 332a, 332b is configured to provide a 360-degree camera view in their respective directions. Camera member 332 can be a wireless camera configured to wirelessly transmit collected image data (e.g., digital video, photos, etc.) to a remote wireless receiver or workstation (not shown). Camera member 332 can include or be operatively coupled to one or more controllers, processors, memory, circuitry, etc. and/or any number of additional sensors that may facilitate operation of camera member 332. In aspects, camera member 332 can be wired through, for example, tether 336 and through end effector 300 via anvil and cartridge assemblies 320, 310. Such wiring may extend through surgical stapling apparatus 10 to a controller (e.g., within a handle housing thereof) and/or may be electrically coupled to a separate or remote workstation (not shown). In aspects, camera member 332 may be in the form of a pill camera. For more detailed description of similar pill cameras, one or more components of which can be included, combined, and/or modified for use with camera member 332, reference can be made to U.S. Patent No. RE41807 to Yokoi et al..

Housing assembly 334 of camera assembly 330 includes a proximal housing 334a and a distal housing 334b that are threadedly coupled together to enable proximal and distal housings 334a, 334b to be selectively separated from one another for accessing camera member 332. Although shown as threadedly coupled together, proximal and distal housings 334a, 334b can be secured together using any suitable mechanical interface (e.g., friction fit, magnetics, adhesive, etc.). Further, although housing assembly 334 is shown with an oblong, egg-shaped configuration, housing assembly 334 can have any suitable geometry. In some aspects, proximal and distal housings 334a, 334b may be integrally (e.g., monolithically) formed. In aspects, one or both proximal and distal housings 334a, 334b may include transparent material to facilitate imaging of adjacent tissue "T" (e.g., anastomosed tissue "AT"; see FIG. 6) by camera member 332.

Turning now to FIGS. 4-7, in operation, once end effector 300 of the surgical stapling apparatus 10 is positioned adjacent to a surgical site with camera assembly 330 and anvil assembly 320 distal of the tissue "T" to be fastened and cartridge assembly 310 proximal to the tissue "T" to be fastened, anvil assembly 320 of end effector 300 can be approximated toward cartridge assembly 310 of end effector 300 to grasp the tissue "T" between cartridge and anvil assemblies 310, 320. Stapling apparatus 10 can then be fired for firing fasteners "F" into tissue "T" and cutting the tissue "T" with a knife (not shown) of stapling apparatus 10. For a more detailed description of firing, cutting, and/or fastening of fasteners "F" and/or replacement of cartridge and/or anvil assemblies 310, 320, reference can be made to U.S. Patent No. 7,303,106.

Anvil assembly 320 of end effector 300 can then be separated from cartridge assembly 310 of end effector 300 to release the fastened and now anastomosed tissue "AT." With camera 332 disposed distal to the anastomosed tissue "AT," proximal camera 332a can be utilized to generate image data of the distal portion of the anastomosed tissue "AT." Such image data (e.g., photos, videos, etc.) can be electrically communicated (e.g., wirelessly) to a display (not shown, but which may be on a handle of surgical stapling apparatus 10 and/or coupled to a remote workstation) so that a clinician can inspect a distal portion of the staple formation and hemostasis of the anastomosed tissue "AT." End effector 300 can be retracted proximally to a position in which camera member 332 of camera assembly 330 is proximal to the anastomosed tissue "AT" so that distal camera 332a of camera member 332 can be utilized to generate image data of the proximal portion of the anastomosed tissue "AT." Again, such image data can be electrically communicated (e.g., wirelessly) to the display so that a clinician can inspect the proximal portion of the staple formation and hemostasis of the anastomosed tissue "AT."

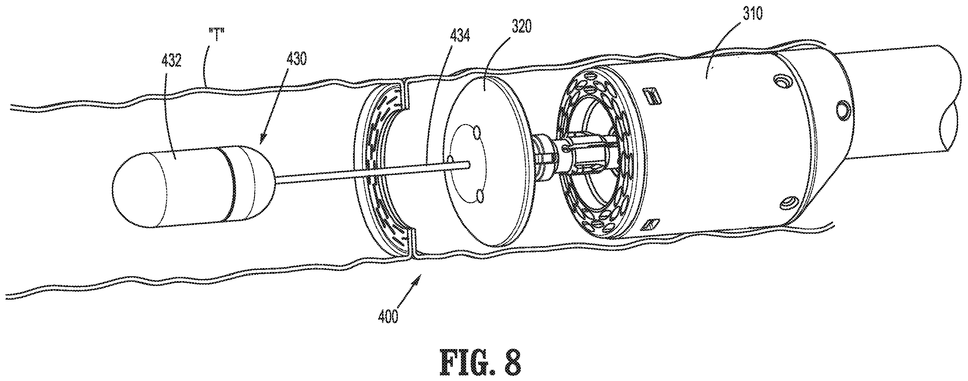

As seen in FIG. 8, another end effector of surgical stapling apparatus 10 is generally referred to as 400. End effector 400 is similar to end effector 300, but does not include a housing assembly 330. In particular, end effector 400 includes a cartridge assembly 310, an anvil assembly 320 movably coupled to cartridge assembly 310 between clamped and unclamped positions, and a camera assembly 430. Camera assembly 430 includes a camera member 432 and a tether 434 that couples camera member 432 to anvil assembly 320. Tether 434 has a proximal end portion coupled to anvil assembly 320 and a distal end portion coupled to a proximal end portion of camera member 432.

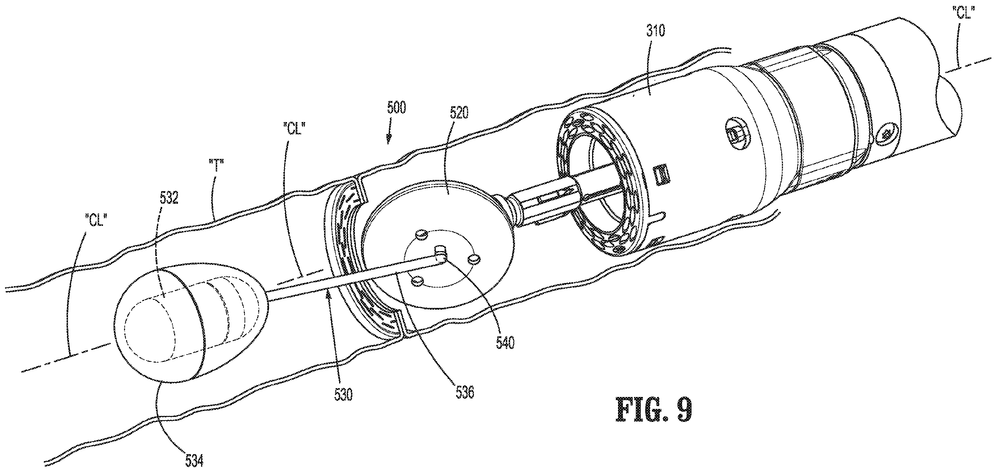

With reference to FIG. 9, yet another end effector of surgical stapling apparatus is generally referred to as 500. End effector 500 is substantially similar to end effector 300 and includes a cartridge assembly 310, an anvil assembly 520 moveably coupled to cartridge assembly 310 between clamped and unclamped positions while in an untilted orientation, and a camera assembly 530. Anvil assembly 520 is configured to be positioned in a tilted orientation after a firing of end effector 500 as described in greater detail in U.S. Patent No. 8,109,426 to Milliman et al.. Camera assembly 530 includes a camera member 532, a housing assembly 534 supporting camera member 532, and a tether 536 that connects housing assembly 534 to anvil assembly 520. End effector 500 further includes a ball joint 540 that pivotably couples to a proximal end portion of tether 536 to a distal end portion of anvil assembly 520 to facilitate tilting movement of anvil assembly 520 relative to cartridge and camera assemblies 310, 530 and center line "CL" of surgical stapling apparatus 10.

The various aspects disclosed herein may also be configured to work with robotic surgical systems and what is commonly referred to as "Telesurgery." Such systems employ various robotic elements to assist the clinician and allow remote operation (or partial remote operation) of surgical instrumentation. Various robotic arms, gears, cams, pulleys, electric and mechanical motors, etc. may be employed for this purpose and may be designed with a robotic surgical system to assist the clinician during the course of an operation or treatment. Such robotic systems may include remotely steerable systems, automatically flexible surgical systems, remotely flexible surgical systems, remotely articulating surgical systems, wireless surgical systems, modular or selectively configurable remotely operated surgical systems, etc.

The robotic surgical systems may be employed with one or more consoles that are next to the operating theater or located in a remote location. In this instance, one team of clinicians may prep the patient for surgery and configure the robotic surgical system with one or more of the instruments disclosed herein while another clinician (or group of clinicians) remotely controls the instruments via the robotic surgical system. As can be appreciated, a highly skilled clinician may perform multiple operations in multiple locations without leaving his/her remote console which can be both economically advantageous and a benefit to the patient or a series of patients. For a detailed description of exemplary medical work stations and/or components thereof, reference may be made to U.S. Patent Application Publication No. 2012/0116416, and PCT Application Publication No. WO2016/025132.

It should be understood that the disclosed structure can include any suitable mechanical, electrical, and/or chemical components for operating the disclosed system or components thereof. For instance, such electrical components can include, for example, any suitable electrical and/or electromechanical, and/or electrochemical circuitry, which may include or be coupled to one or more printed circuit boards. As appreciated, the disclosed computing devices (and/or servers), such as the disclosed camera 332, can include, for example, a "controller," "processor," "digital processing device" and like terms, and which are used to indicate a microprocessor or central processing unit (CPU). The CPU is the electronic circuitry within a computer that carries out the instructions of a computer program by performing the basic arithmetic, logical, control and input/output (I/O) operations specified by the instructions, and by way of non-limiting examples, include server computers. In some aspects, the controller includes an operating system configured to perform executable instructions. The operating system is, for example, software, including programs and data, which manages hardware of the disclosed apparatus and provides services for execution of applications for use with the disclosed apparatus. Those of skill in the art will recognize that suitable server operating systems include, by way of non-limiting examples, FreeBSD, OpenBSD, NetBSD^{®}, Linux, Apple^{®} Mac OS X Server^{®}, Oracle^{®} Solaris^{®}, Windows Server^{®}, and Novell^{®} NetWare^{®}. In some aspects, the operating system is provided by cloud computing.

In some aspects, the term "controller" may be used to indicate a device that controls the transfer of data from a computer or computing device to a peripheral or separate device and vice versa, and/or a mechanical and/or electromechanical device (e.g., a lever, knob, etc.) that mechanically operates and/or actuates a peripheral or separate device.

In aspects, the controller includes a storage and/or memory device. The storage and/or memory device is one or more physical apparatus used to store data or programs on a temporary or permanent basis. In some aspects, the controller includes volatile memory and requires power to maintain stored information. In various aspects, the controller includes non-volatile memory and retains stored information when it is not powered. In some aspects, the non-volatile memory includes flash memory. In certain aspects, the non-volatile memory includes dynamic random-access memory (DRAM). In some aspects, the non-volatile memory includes ferroelectric random-access memory (FRAM). In various aspects, the non-volatile memory includes phasechange random access memory (PRAM). In certain aspects, the controller is a storage device including, by way of non-limiting examples, CD-ROMs, DVDs, flash memory devices, magnetic disk drives, magnetic tapes drives, optical disk drives, and cloud-computing-based storage. In various aspects, the storage and/or memory device is a combination of devices such as those disclosed herein.

In various aspects, the memory can be random access memory, read-only memory, magnetic disk memory, solid state memory, optical disc memory, and/or another type of memory. In various aspects, the memory can be separate from the controller and can communicate with the processor through communication buses of a circuit board and/or through communication cables such as serial ATA cables or other types of cables. The memory includes computer-readable instructions that are executable by the processor to operate the controller. In various aspects, the controller may include a wireless network interface to communicate with other computers or a server. In aspects, a storage device may be used for storing data. In various aspects, the processor may be, for example, without limitation, a digital signal processor, a microprocessor, an ASIC, a graphics processing unit ("GPU"), field-programmable gate array ("FPGA"), or a central processing unit ("CPU").

The memory stores suitable instructions and/or applications, to be executed by the processor, for receiving the sensed data (e.g., sensed data from camera), accessing storage device of the controller, generating a raw image based on the sensed data, comparing the raw image to a calibration data set, identifying an object based on the raw image compared to the calibration data set, transmitting object data to a post-processing unit, and displaying the object data to a graphic user interface. Although illustrated as part of the disclosed structure, it is also contemplated that a controller may be remote from the disclosed structure (e.g., on a remote server), and accessible by the disclosed structure via a wired or wireless connection. In aspects where the controller is remote, it is contemplated that the controller may be accessible by, and connected to, multiple structures and/or components of the disclosed system.

The term "application" may include a computer program designed to perform particular functions, tasks, or activities for the benefit of a user. Application may refer to, for example, software running locally or remotely, as a standalone program or in a web browser, or other software which would be understood by one skilled in the art to be an application. An application may run on the disclosed controllers or on a user device, including for example, on a mobile device, an IOT device, or a server system.

In some aspects, the controller includes a display to send visual information to a user. In various aspects, the display is a cathode ray tube (CRT). In various aspects, the display is a liquid crystal display (LCD). In certain aspects, the display is a thin film transistor liquid crystal display (TFT-LCD). In aspects, the display is an organic light emitting diode (OLED) display. In certain aspects, on OLED display is a passive-matrix OLED (PMOLED) or active-matrix OLED (AMOLED) display. In aspects, the display is a plasma display. In certain aspects, the display is a video projector. In various aspects, the display is interactive (e.g., having a touch screen) that can detect user interactions/gestures/responses and the like. In some aspects, the display is a combination of devices such as those disclosed herein.

The controller may include or be coupled to a server and/or a network. As used herein, the term "server" includes "computer server," "central server," "main server," and like terms to indicate a computer or device on a network that manages the disclosed apparatus, components thereof, and/or resources thereof. As used herein, the term "network" can include any network technology including, for instance, a cellular data network, a wired network, a fiberoptic network, a satellite network, and/or an IEEE 802.11a/b/g/n/ac wireless network, among others.

In various aspects, the controller can be coupled to a mesh network. As used herein, a "mesh network" is a network topology in which each node relays data for the network. All mesh nodes cooperate in the distribution of data in the network. It can be applied to both wired and wireless networks. Wireless mesh networks can be considered a type of "Wireless ad hoc" network. Thus, wireless mesh networks are closely related to Mobile ad hoc networks (MANETs). Although MANETs are not restricted to a specific mesh network topology, Wireless ad hoc networks or MANETs can take any form of network topology. Mesh networks can relay messages using either a flooding technique or a routing technique. With routing, the message is propagated along a path by hopping from node to node until it reaches its destination. To ensure that all its paths are available, the network must allow for continuous connections and must reconfigure itself around broken paths, using self-healing algorithms such as Shortest Path Bridging. Self-healing allows a routing-based network to operate when a node breaks down or when a connection becomes unreliable. As a result, the network is typically quite reliable, as there is often more than one path between a source and a destination in the network. This concept can also apply to wired networks and to software interaction. A mesh network whose nodes are all connected to each other is a fully connected network.

In some aspects, the controller may include one or more modules. As used herein, the term "module" and like terms are used to indicate a self-contained hardware component of the central server, which in turn includes software modules. In software, a module is a part of a program. Programs are composed of one or more independently developed modules that are not combined until the program is linked. A single module can contain one or several routines, or sections of programs that perform a particular task.

As used herein, the controller includes software modules for managing various aspects and functions of the disclosed system or components thereof.

The disclosed structure may also utilize one or more controllers to receive various information and transform the received information to generate an output. The controller may include any type of computing device, computational circuit, or any type of processor or processing circuit capable of executing a series of instructions that are stored in memory. The controller may include multiple processors and/or multicore central processing units (CPUs) and may include any type of processor, such as a microprocessor, digital signal processor, microcontroller, programmable logic device (PLD), field programmable gate array (FPGA), or the like. The controller may also include a memory to store data and/or instructions that, when executed by the one or more processors, cause the one or more processors to perform one or more methods and/or algorithms.

The phrases "in an aspect," "in aspects," "in various aspects," "in some aspects," or "in other aspects" may each refer to one or more of the same or different aspects in accordance with the present disclosure. A phrase in the form "A or B" means "(A), (B), or (A and B)." A phrase in the form "at least one of A, B, or C" means "(A); (B); (C); (A and B); (A and C); (B and C); or (A, B, and C)."

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques).

Certain aspects of the present disclosure may include some, all, or none of the above advantages and/or one or more other advantages readily apparent to those skilled in the art from the drawings, descriptions, and claims included herein. Moreover, while specific advantages have been enumerated above, the various aspects of the present disclosure may include all, some, or none of the enumerated advantages and/or other advantages not specifically enumerated above.

The aspects disclosed herein are examples of the disclosure and may be embodied in various forms. For instance, although certain aspects herein are described as separate, each of the aspects herein may be combined with one or more of the other aspects herein. Specific structural and functional details disclosed herein are not to be interpreted as limiting, but as a basis for the claims and as a representative basis for teaching one skilled in the art to variously employ the present disclosure in virtually any appropriately detailed structure. Like reference numerals may refer to similar or identical elements throughout the description of the figures.

Any of the herein described methods, programs, algorithms, or codes may be converted to, or expressed in, a programming language or computer program. The terms "programming language" and "computer program," as used herein, each include any language used to specify instructions to a computer, and include (but is not limited to) the following languages and their derivatives: Assembler, Basic, Batch files, BCPL, C, C+, C++, Delphi, Fortran, Java, JavaScript, machine code, operating system command languages, Pascal, Perl, PL1, scripting languages, Visual Basic, metalanguages which themselves specify programs, and all first, second, third, fourth, fifth, or further generation computer languages. Also included are database and other data schemas, and any other meta-languages. No distinction is made between languages which are interpreted, compiled, or use both compiled and interpreted approaches. No distinction is made between compiled and source versions of a program. Thus, reference to a program, where the programming language could exist in more than one state (such as source, compiled, object, or linked) is a reference to any and all such states. Reference to a program may encompass the actual instructions and/or the intent of those instructions.

As can be appreciated, securement of any of the components of the disclosed systems can be effectuated using known securement techniques such welding, crimping, gluing, fastening, etc.

Persons skilled in the art will understand that the structures and methods specifically described herein and illustrated in the accompanying figures are non-limiting exemplary aspects, and that the description, disclosure, and figures should be construed merely as exemplary of particular aspects. It is to be understood, therefore, that the present disclosure is not limited to the precise aspects described, and that various other changes and modifications may be effected by one skilled in the art without departing from the scope or spirit of the disclosure. Additionally, it is envisioned that the elements and features illustrated or described in connection with one exemplary aspect may be combined with the elements and features of another without departing from the scope of the present disclosure, and that such modifications and variations are also intended to be included within the scope of the present disclosure. Indeed, any combination of any of the presently disclosed elements and features is within the scope of the present disclosure. Accordingly, the subject matter of the present disclosure is not to be limited by what has been particularly shown and described.

## Claims

1. A surgical stapling apparatus (10), comprising:
a cartridge assembly (310) supporting an array of fasteners therein;
an anvil assembly (320, 520) movably positioned relative to the cartridge assembly between an unclamped position and a clamped position for forming the array of fasteners; and
a camera assembly (330, 430, 530) coupled to the anvil assembly and positioned to collect and transmit image data of a fastened tissue site wherein the camera assembly is coupled to the anvil assembly via a tether (336, 434, 536) and wherein the camera assembly includes a camera member (332, 432, 532) including a distal camera (332b) supported on a distal end portion of the camera member
**characterised in that**
the camera member further includes a proximal camera (332a) supported on a proximal end portion of the camera member.

2. The surgical stapling apparatus of claim 1, wherein the camera member is wireless and supported in a housing assembly (334, 534).

3. The surgical stapling apparatus of claim 2, wherein the housing assembly includes a first portion (334a) and a second portion (334b) that are removably coupled to one another.

4. The surgical stapling apparatus of claim 3, wherein the first and second portions of the housing assembly are threadedly coupled together.

5. The surgical stapling apparatus of claim 4, wherein the tether is coupled to the anvil assembly by a ball joint (540).

6. The surgical stapling apparatus of claim 5, wherein the tether is coupled to a proximal end portion of the second portion of the housing assembly to maintain the camera member a predetermined distance away from a distal end portion of the anvil assembly.

7. The surgical stapling apparatus of claim 6, wherein each of the proximal and distal cameras is configured to provide a 360-degree view and wirelessly transmit image data of the 360-degree view.

## Patentansprüche

1. Chirurgisches Klammernahtgerät (10), umfassend:
eine Magazinbaugruppe (310), die eine Anordnung von Befestigungselementen darin stützt,
eine Ambossbaugruppe (320, 520), die zum Bilden der Anordnung von Befestigungselementen zwischen einer nicht geklemmten Position und einer geklemmten Position bezüglich der Magazinbaugruppe beweglich positioniert ist, und
eine Kamerabaugruppe (330, 430, 530), die an die Ambossbaugruppe gekoppelt und dazu positioniert ist, Bilddaten einer befestigten Gewebestelle zu sammeln und zu senden, wobei die Kamerabaugruppe über ein Halteband (336, 434, 536) an die Ambossbaugruppe gekoppelt ist und wobei die Kamerabaugruppe ein Kameraglied (332, 432, 532) aufweist, das eine distale Kamera (332b) aufweist, die auf einem distalen Endabschnitt des Kameraglieds gestützt ist,
**dadurch gekennzeichnet, dass**
das Kameraglied ferner eine proximale Kamera (332a) aufweist, die auf einem proximalen Endabschnitt des Kameraglieds gestützt ist.

2. Chirurgisches Klammernahtgerät nach Anspruch 1, wobei das Kameraglied drahtlos ist und in einer Gehäusebaugruppe (334, 534) gestützt ist.

3. Chirurgisches Klammernahtgerät nach Anspruch 2, wobei die Gehäusebaugruppe einen ersten Abschnitt (334a) und einen zweiten Abschnitt (334b) aufweist, die lösbar aneinander gekoppelt sind.

4. Chirurgisches Klammernahtgerät nach Anspruch 3, wobei der erste und der zweite Abschnitt der Gehäusebaugruppe aneinander gewindegekoppelt sind.

5. Chirurgisches Klammernahtgerät nach Anspruch 4, wobei das Halteband durch ein Kugelgelenk (540) an die Ambossbaugruppe gekoppelt ist.

6. Chirurgisches Klammernahtgerät nach Anspruch 5, wobei das Halteband an einen proximalen Endabschnitt des zweiten Abschnitts der Gehäusebaugruppe gekoppelt ist, um das Kameraglied in einem vorbestimmten Abstand von einem distalen Endabschnitt der Ambossbaugruppe weg zu halten.

7. Chirurgisches Klammernahtgerät nach Anspruch 6, wobei die proximale und die distale Kamera jeweils dazu ausgestaltet sind, eine 360-Grad-Ansicht bereitzustellen und Bilddaten der 360-Grad-Ansicht drahtlos zu senden.

## Revendications

1. Appareil d'agrafage chirurgical (10), comprenant :
un ensemble cartouche (310) supportant un réseau d'éléments de fixation à l'intérieur ;
un ensemble enclume (320, 520) positionné de manière mobile par rapport à l'ensemble cartouche entre une position non serrée et une position serrée pour former le réseau d'éléments de fixation ; et
un ensemble caméra (330, 430, 530) couplé à l'ensemble enclume et positionné pour collecter et transmettre des données d'image d'un site tissulaire fixé, l'ensemble caméra étant couplé à l'ensemble enclume par l'intermédiaire d'une attache (336, 434, 536), et l'ensemble caméra comprenant un élément de caméra (332, 432, 532) comprenant une caméra distale (332b) supportée sur une partie d'extrémité distale de l'élément de caméra **caractérisé en ce que**
l'élément de caméra comprend en outre une caméra proximale (332a) supportée sur une partie d'extrémité proximale de l'élément de caméra.

2. Appareil d'agrafage chirurgical selon la revendication 1, l'élément de caméra étant sans fil et supporté dans un ensemble boîtier (334, 534).

3. Appareil d'agrafage chirurgical selon la revendication 2, l'ensemble boîtier comprenant une première partie (334a) et une seconde partie (334b) qui sont couplées de manière amovible l'une à l'autre.

4. Appareil d'agrafage chirurgical selon la revendication 3, les première et seconde parties de l'ensemble boîtier étant accouplées ensemble par filetage.

5. Appareil d'agrafage chirurgical selon la revendication 4, l'attache étant couplée à l'ensemble enclume par un joint à rotule (540).

6. Appareil d'agrafage chirurgical selon la revendication 5, l'attache étant couplée à une partie d'extrémité proximale de la seconde partie de l'ensemble boîtier pour maintenir l'élément de caméra à une distance prédéterminée d'une partie d'extrémité distale de l'ensemble enclume.

7. Appareil d'agrafage chirurgical selon la revendication 6, chacune des caméras proximale et distale étant configurée pour fournir une vue à 360 degrés et transmettre sans fil des données d'image de la vue à 360 degrés.
